# EUROPÄISCHE PATENTSCHRIFT

(11) **EP 1 641 459 B1**
(45) Veröffentlichungstag und Bekanntmachung des Hinweises auf die Patenterteilung: **23.01.2013**
(21) Anmeldenummer: 04740505.5
(22) Anmeldetag: 01.07.2004
(51) Int. Cl.: A61K 31/505, A61K 31/70, A61K 31/662, A61K 31/665, A61K 31/683, A61P 17/00, A61K 35/74

(54) **VERWENDUNG VON AUS EXTREMOPHILEN BAKTERIEN GEWONNENEN OSMOLYTEN ZUR HERSTELLUNG VON ARZNEIMITTELN ZUR ÄUSSERLICHEN BEHANDLUNG DER NEURODERMITIS**
USE OF OSMOLYTES OBTAINED FROM EXTREMOPHILIC BACTERIA FOR PRODUCING MEDICAMENTS FOR THE EXTERNAL TREATMENT OF NEURODERMATITIS
UTILISATION D'OSMOLYTES OBTENUS A PARTIR DE BACTERIES EXTREMOPHILES POUR PRODUIRE DES MEDICAMENTS PERMETTANT DE TRAITER DE MANIERE EXTERNE UNE NEVRODERMITE

(30) Priorität: 03.07.2003 DE 10330243
(43) Veröffentlichungstag der Anmeldung: 05.04.2006
(73) Patentinhaber: Bitop Aktiengesellschaft Für Biotechnische Optimierung, 58453 Witten (DE)
(72) Erfinder: KRUTMANN, Jean, 41844 Wegberg (DE)
(74) Vertreter: Schneiders, Josef
(86) Internationale Anmeldenummer: PCT/EP2004/007134
(87) Internationale Veröffentlichungsnummer: WO 2005/002581

(56) Entgegenhaltungen:
- WO-A-01/76572
- WO-A-02/15866
- WO-A-02/15868
- WO-A-02/072583
- DE-A- 10 040 933
- DE-A- 10 133 202
- DE-A- 19 933 461
- JP-A- 2002 265 327
- US-B1- 6 267 973
- SYMRISE / HAARMANN & REIMER GMBH ET AL: "N-Acyl-hydroxyaminosÃ ureester in kosmetischen und dermatologischen Zubereitungen" RESEARCH DISCLOSURE, KENNETH MASON PUBLICATIONS, HAMPSHIRE, GB, Bd. 468, Nr. 117, April 2003 (2003-04), XP007132559 ISSN: 0374-4353
- NIWA YUKIE: "Antioxidant ointment consisting of treated natural materials and very low concentrations of glucocorticoid shows remarkable effectiveness in atopic dermatitis" OYO YAKURI, Bd. 58, Nr. 2, Dezember 1999 (1999-12), Seiten 35-44, XP009039638 ISSN: 0300-8533
- HANIFIN J M ET AL: "Effects of a low-potency corticosteroid lotion plus a moisturizing regimen in the treatment of atopic dermatitis" CURRENT THERAPEUTIC RESEARCH - CLINICAL AND EXPERIMENTAL 1998 UNITED STATES, Bd. 59, Nr. 4, 1998, Seiten 227-233, XP001203746 ISSN: 0011-393X
- SYDLIK ULRICH ET AL: "The compatible solute ectoine protects against nanoparticle-induced neutrophilic lung inflammation", TOXICOLOGY LETTERS (SHANNON), vol. 189, no. Sp. Iss. SI, September 2009 (2009-09), page S188, & 46TH CONGRESS OF THE EUROPEAN-SOCIETIES-OF-TOXICOLOGY; DREDEN, GERMANY; SEPTEMBER 13 -16, 2009 ISSN: 0378-4274
- SCHNOPP C H ET AL: "VOM OELBAD BIS ZUM LACTOBACILLUS WIE BEHANDELT MAN NEURODERMITIS?//FROM OIL BATH TO LACTOBACILLUS. HOW TO TREAT NEURODERMATITIS?", FORTSCHRITT DER MEDIZIN MMW ORIGINALIA, URBAN UND VOGEL MEDIEN UND MEDIZIN VERLAGSGESELLSCHAFT, DE, vol. 144, no. 10, 7 March 2002 (2002-03-07), pages 33-38, XP009055644, ISSN: 1438-3276

## Beschreibung

Extremophile Bakterien sind außergewöhnliche Mikroorganismen, die fähig sind, unter extremen Bedingungen, z.B. bei biologisch extremen Salzkonzentrationen bis 200 g Kochsalz pro Liter und bei Temperaturen im Bereich von 60 bis 110° C zu leben und sich zu vermehren. Solche Lebensbedingungen würden bei normalen (mesophilen) Organismen zum sofortigen Tod und mindestens zu massiven Schädigungen zellulärer Strukturen führen.

In den letzten Jahren wurde daher ein großer Forschungsaufwand betrieben, um diejenigen biochemischen Komponenten zu identifizieren, auf welche die bemerkenswerte thermische, chemische und physikalische Stabilität der Zellstrukturen extremophiler Organismen zurückzuführen ist.

Zu der hohen Temperaturstabilität von Zellstrukturen tragen in erheblichem Maß niedermolekulare organische Substanzen im intrazellulären Milieu bei, die als Osmolyte oder kompatible Solute bezeichnet werden. Die in den extremophilen Mikroorganismen gefundenen Osmolyte werden von menschlichen oder tierischen Zellen nicht gebildet. In neuerer Zeit konnten in extremophilen Mikroorganismen erstmals verschiedene neuere Osmolyte identifiziert werden. Hierzu gehören beispielsweise Ectoin, Hydroxyectoin, Firoin, Firoin-A, Diglycerolphosphat, cyclisches Diphosphoglycerat, 1,3-Di-mannosyl-myo-inositol-phosphat (DMIP) und Diinositolphosphat. Alle werden aus extremophilen Mikroorganismen gewonnen und aufgearbeitet bzw. gereinigt (vgl. EP-A 94 903 874; EP-A 98 121 243; DE-A 100 47 444) und bilden eine bekannte Gruppe niedermolekularer Stoffe mit Schutzeigenschaften für ansonsten sensible Zellen. In einigen Fällen konnte der Beitrag dieser Verbindungen zum Schutz von Hautzellen gegenüber externen Stressbedingungen wie Hitze- und Trockenheit auf dem Gebiet der Kosmetik ( vgl. US-A 6 267 973, ) bereits gezeigt werden.

Verschiedentlich wurde auch schon vorgeschlagen, vorzugsweise topische Arzneimittel zum Schutz der Haut vor externen Stresseinwirkungen oder zur Behandlung von Erkrankungen einzusetzen, die durch den enzymatischen Abbau von Gewebestrukturen verursacht werden ( vgl. DE-A 100 06 578). Aufgeführt wurden neben anderen Krankheiten allgemein Erkrankungen des Immunsystems, Autoimmunerkrankungen, Entzündungsprozesse sowie akute und chronische Entzündungen.

Die ebenfalls auf die Verwendung von Ectoin und anderen Osmolyten gerichtete DE-A 198 34 816 betrifft Kosmetika mit einer Schutzwirkung gegen die UV-Bestrahlung der Haut, die daneben auch eine Wirksamkeit bei der Stabilisierung von Nukleinsäuren menschlicher Hautzellen aufweisen sollen.

Das Osmolyt Ectoin wurde auch als Feuchtigkeitsspender (moisturizer) in kosmetischen Produkten eingesetzt, mit dem Ziel, die gesunde, klinisch unauffällige menschliche Haut gegen schädigende Wirkungen der ultravioletten Sonnenstrahlung zu schützen (EP-A 19 990 941). Aufgrund von in-vitro und in-vivo Untersuchungen wird angenommen, dass die kosmetische Wirkung von Ectoin unter anderem darauf beruht, dass die Funktionen von epidermalen Langerhanszellen, sowie von epidermalen Keratinozyten und dermalen Fibroblasten dahingehend beeinflusst werden, dass sie gegen proentzündliche Wirkungen der UV-Strahlung, die z.B. zur Ausbildung eines Sonnenbrandes führen würden, besser geschützt sind.

Die Herstellung eines Arzneimittels zur allgemeinen Behandlung. von Hauterkrankungen mittels Osmolyten, insbesondere Ectoin oder Hydroxyectoin ist aus der EP-A 0 887 418 bekannt, die von der Anmelderin Bitop AG, seinerzeit selbst hinterlegt wurde. Hierbei ging man davon aus, dass diese Wirkstoffe zur Stabilisierung von Enzymen und anderen Biomolekülen beitragen und in der Folge die Stabilisierung denaturierender Bedingungen unterstützen können.

In den Offenlegungsschriften DE-A 199 33 460, DE-A 199 33 461, DE-A 199 33 463 und DE-A 199 33 466 ist vorgeschlagen worden, Ectoine aufgrund einer antioxidativen Wirkung als Radikalfänger einzusetzen, und damit die Haut insbesondere vor der durch Sonnenbestrahlung beschleunigten und verstärkten Hautalterung zu schützen. Damit sollten auch unerwünschte Zustände der Haut, die aus oxidativen Vorgängen resultieren, vermieden werden. Die WO 01/72287 beschreibt unter ähnlichen Prämissen wie in den zuletzt genannten Druckschriften vorgeschlagen, die Anwendung von Ectoinen bei der Behandlung von UV-induzierter Immunsuppression.

Die Neurodermitis, auch endogenes Ekzem oder atopische Dermatitis genannt, ist noch immer eine sehr häufige und eine mangels geeigneter Behandlungsmethoden meist über Jahre oder Jahrzehnte verlaufende Hautkrankheit, die durch dauernden und kaum wirksam zu bekämpfenden Juckreiz bei den Patienten zu enormem Leidensdruck führt. Eine der angenommenen Ursachen hierfür ist eine übermäßig trockene und empfindliche Haut mit starker Entzündungsneigung. Die Krankheit beginnt sehr häufig schon in der Kindheit oder frühen Jugend. Nach neueren Untersuchungen können bis zu 12 % eines Jahrgangs betroffen sein.

Klinisch kommt es zur Entwicklung relativ unscharf begrenzter, papulöser, nässender, schuppender, exkoriierter, zum Teil superinfizierter ekzematöser Hautveränderungen, die sich primär im Gesicht, im Decolleté, am Hals und an den großen Gelenkbeugen manifestieren, aber auch das gesamte Integument betreffen können. Im Falle einer Chronifizierung ist zudem häufig eine Lichenifikation der Hautveränderungen zu beobachten. Das sogenannte Säuglingsekzem tritt oft schon im dritten Lebensmonat auf. Viele Erkrankungen findet man im Spiel- und Schulalter. Besonders Kinder kratzen die betroffenen Hautstellen immer wieder auf und provozieren damit ungewollte schwer zu behandelnde Superinfektionen.

Über die möglichen Ursachen dieser schweren Erkrankung ist noch nichts Sicheres bekannt. Die Ansichten der Fachleute hierüber sind uneinheitlich. Neben anderen, noch nicht genau geklärten Faktoren, spielt wohl auch eine Störung im Abwehrsystem des Körpers eine Rolle bei der Krankheitsentstehung. Es wird auch vermutet, dass erbliche Belastung den Ausbruch der Krankheit zumindest fördert. Es ist auch beobachtet worden, dass an Neurodermitis Erkrankte auf an sich unschädliche Umweltstoffe allergisch reagieren.

Notgedrungen richtete sich die Behandlung bisher mehr auf die Symptome, insbesondere die Behandlung der unangenehmen Hauterscheinungen und des Juckreizes. Üblich ist die topische Behandlung mit Glukokortikosteroiden oder Calcineurininhibitoren und/oder eine gesteuerte Bestrahlung mit ultraviolettem Licht. In Ermangelung einer kurativen Therapie setzen die zur Zeit angewandten symptomatischen Behandlungsstrategien an verschiedenen, sich ergänzenden Punkten an. Beispielsweise wird wegen der Hauttrockenheit und offensichtlicher Mängel im Fettstoffwechsel der Haut neben kühlenden Umschlägen, aber auch mit fettenden Salben oder Ölbädern behandelt. Hierzu gehört beispielsweise die Behandlung mit hochdosiertem Nachtkerzensamenöl (EPOGAM^{®}- Kapseln). Damit soll der Haut über den Stoffwechsel Gamma-Linolensäure zugeführt werden. Bei der Behandlung der entzündeten Hautstellen werden antiinflammatorische Wirkstoffe eingesetzt. Antihistaminika sollen den Juckreiz mildern. Cortisonpräparate lindern zwar auftretende Hautreizungen, führen aber nicht unmittelbar zur Heilung der Krankheit. Antibiotika können bei der Behandlung infizierter Stellen der Haut angezeigt sein.

Aus dem oben abgehandelten Stand der Technik wird deutlich, dass sich für die Behandlung der Neurodermitis noch kein bestimmtes Behandlungsschema durchgesetzt hat und im Einzelfall je nach Krankheitsbild entschieden werden muss, welche der oben genannten Methoden und welche der vielfältigen Kombinationsmöglichkeiten Erfolg versprechen könnten. Jeder Fortschritt und jede neuartige Alternative in der Therapie muss daher begrüßt werden, insbesondere solange sich die Wissenschaft über die eigentlichen Ursachen dieser schweren Krankheit noch nicht einig ist und bisher kein einziges vorgeschlagenes Mittel einen durchgreifenden Erfolg gezeigt hat. Bei sorgfältiger Abwägung der bekannten Therapiemaßnahmen kann lediglich davon ausgegangen werden, dass als Einzeimaßnahme die Behandlung mit Glukokortikosteroiden als die bisher wirksamste, aber auch nebenwirkungsreichste Therapie betrachtet wird. Vergleichbar erfolgreich ist die Therapie mit Calcineurininhibitoren, welche jedoch bei gleichzeitiger Bestrahlung mit UV- bzw. Sonnenlicht den Nachteil eines gewissen Krebsrisikos in sich birgt.

Ziel der Erfindung ist es, ein in der Wirksamkeit mit den Glukokortikoiden oder Calcineurininhibitoren vergleichbares aber mit weniger Nebenwirkungen behaftetes und sichereres Mittel zur Behandlung der Neurodermitis bereitzustellen.

Es wurde nun gefunden, dass die topische Applikation von Osmolyten, insbesondere von Ectoin und Hydroxyectoin, bei der Behandlung der Neurodermitis eine überraschend hohe Wirksamkeit aufweist.

Im Vergleich mit der Placebobehandlung wird bei der Behandlung mit Osmolyten, insbesondere Ectoin und Hydroxyectoin, ein unerwartet stark beschleunigtes Abheilen betroffener Hautveränderungen erzielt. Neben dem schnellen Wirkungseintritt bedeutet dies insbesondere bei langfristiger Anwendung einen erheblichen medizinischen Fortschritt, weil die bekannten und zahlreichen Nebenwirkungen der Glukokortikoide durch die Erfindung praktisch ganz vermieden werden können. Da die Osmolyten der Erfindung nach derzeitiger Kenntnis praktisch nebenwirkungsfrei sind, bedeutet dies auch einen wichtigen Fortschritt in der Arzneimittelsicherheit.
Bemerkenswert ist auch, dass die Wirksamkeit eines Calcineurininhibitors durch die erfindungsgemäß vorgeschlagenen Mitteln deutlich übertroffen werden konnte. Von Calcineurininhibitoren ist, wie erwähnt, bei gleichzeitiger Sonnenexposition oder Bestrahlung mit künstlichem UV-Licht allgemein ein erhöhtes Hautkrebsrisiko bekannt. Durch die Erfindung kann nunmehr ein Mittel bereitgestellt werden, das dieses Risiko nicht aufweist und sogar wirksamer ist.

Gegenstand der Erfindung ist die Verwendung von Osmolyten, sowie von deren Derivaten und/oder pharmakologisch verträglichen Salzen zur Herstellung von dermatologischen Zubereitungen zur topischen Prophylaxe, Behandlung und/oder Pflege bei der Neurodermitis.

Einige der erfindungsgemäßen Wirkstoffe sind schwache Basen oder Säuren und können daher auch, in manchen Fällen sogar bevorzugt, in ihrer pharmakologisch besonders verträglichen neutralen Salzform zum Einsatz kommen.

Als pharmakologisch verträgliche Salze kommen die Alkali- oder Erdalkalisalze, insbesondere Kalium, Natrium, Magnesium und Calcium, aber auch Salze mit organischen Basen wie z.B. mit nicht toxischen aliphatischen oder aromatischen Aminen in Frage.

Überwiegt im Falle der Anwesenheit von Stickstoffatomen im Wirkstoffmolekül die basische Natur, werden Salze mit pharmakologisch unbedenklichen organischen oder anorganischen Säuren, wie z.B. Essigsäure, Citronensäure, Weinsäure, Mandelsäure, Äpfelsäure, Milchsäure, Chlorwasserstoffsäure, Bromwasserstoffsäure, Schwefelsäure oder Phosphorsäure gebildet.

Bevorzugte Osmolyten sind Ectoin, Hydroxyectoin sowie deren gleichwirkende Derivate und Salze. Ebenfalls bevorzugt sind Kombinationspräparate, die einen der Wirkstoffe oder beide Wirkstoffe nebeneinander ggf. mit weiteren Wirkstoffen enthalten. Gleichwirkende Derivate sind Verbindungen, die sich durch strukturelle Abweichungen insbesondere der funktionellen Gruppen und der Substituenten von den oben genannten Grundstrukturen der Osmolyten unterscheiden, aber im Sinne der Erfindung gleichwirkend sind. Im Falle des Hydroxyectoins können beispielsweise aus der Hydroxylgruppe mit gesättigten oder ungesättigten, geradkettigen oder verzweigten C¹ bis C⁴ Alkylgruppen entsprechende Alkoxylgruppen gebildet werden. Mit C¹ bis C⁴ Carbonsäuren werden entsprechende Ester gebildet. Aus der Carboxylgruppe entstehen Amide die wiederum gesättigte oder ungesättigte, geradkettige oder verzweigte C¹ bis C⁴ Alkylgruppen am Stickstoffatom aufweisen können. Mit entsprechenden C¹ bis C⁴ - Alkoholen erhält man wirksame Ester. Die Carboxylatgruppe wiederum kann durch eine Carbonyl- , Sulfonyl- oder Sulfonylatgruppe ersetzt werden. Entsprechende Modifikationen der übrigen genannten Osmolyte sind in analoger Weise unter Erhalt oder sogar Verbesserung der Wirkung möglich.

Allgemein können die erfindungsgemäßen Wirkstoffe oder deren Kombinationen unter Einsatz üblicher hautverträglicher und pharmakologisch unbedenklicher Hilfs- und Zusatzstoffe in bekannter Weise galenisch verarbeitet werden. Solche Zusätze sind z. B. Emulgatoren, Lösungsmittel, Verdickungsmittel, Füllstoffe, Stabilisatoren, Konservierungsmittel oder Antioxidantien.
Mit oberflächenaktiven Mitteln, wie Polyoxyethylen-sorbitansäure und Estern oder Salzen der Gallensäure kann ggf. auch die Bioverfügbarkeit verbessert werden. Im vorliegenden Falle ist es jedoch grundsätzlich zu empfehlen, wegen des immer vorhandenen Allergiepotentials und der allgemein hohen Empfindlichkeit erkrankter Haut möglichst wenig Hilfsstoffe einzusetzen.

Neben den erfindungsgemäß vorgeschlagenen Osmolyten können auch weitere für die Behandlung geeignete Wirkstoffe zugesetzt werden. Hierzu gehören beispielsweise Entzündungshemmer, sowie antibakterielle, fungistatische oder fungizide Mittel, sofern diese den erfindungsgemäßen Einsatz aufgrund der oben beschriebenen Gefahr von allergischen Erscheinungen oder der bekannten Empfindlichkeit der meist vorgeschädigten Haut nicht beeinträchtigen.

Zur Einarbeitung unlöslicher Hilfsstoffe werden gewünschtenfalls Dispersionsmittel, wie z. B. Polyacrylate, Lignin, Tannate oder deren Derivate zugesetzt. Als Verdickungsmittel kann z.B. kolloidales Siliciumoxid verwendet werden. Hydrogele lassen sich mit hydrophilen organischen Lösungsmitteln, wie z. B. Glycerin, Glykol oder mit aliphatischen Alkoholen herstellen. Weiterhin ist es im Rahmen der Erfindung möglich, die erfindungsgemäßen Wirkstoffe in Form wirkstoffhaltiger Mikrosome oder Liposome oder als liposomal bzw. mikrosomal verkapselten Wirkstoff mit mikrosomal verkapselten Reparaturenzymen und sog. Actives ggf. neben anderen Hilfs- und weiteren Wirkstoffen zu verwenden.

Die erfindungsgemäßen Wirkstoffe, können zu praktisch allen für die Anwendung an der menschlichen Haut geeigneten Zubereitungsformen verarbeitet werden. Solche Zubereitungsformen sind beispielsweise Tinkturen, Hydrogele, Öl in Wasser-Emulsionen, Wasser in Öl-Emulsionen, Lotionen, Cremes, Salben oder Sprays. Die Konzentration der Wirkstoffe liegt im Normalfall im Bereich von 0,01 bis 20 Gewichtsprozent
Die günstigste Konzentration dürfte im Bereich von 0,1 bis 2 Gewichtsprozent, bezogen auf das Gewicht des eingesetzten Trägermaterials, liegen.

Die folgenden Beispiele dienen zur Erläuterung der Erfindung, beschränken diese jedoch in keiner Weise.

### Beispiel 1

### W/O Emulsion :

| Inhaltsstoff | Gehalt in Gewichts-% |
|---|---|
| Wasser | 85,0 |
| Ectoin | 1,0 |
| Fettgrundlage | 10,3 |
| Mehrwertige Alkohole | 3,2 |
| Feste Verdickungsmittel | 0,3 |
| Konservierungsstoffe | 0,2 |

### Beispiel 2

### W/O Emulsion :

| Inhaltstoff | Gehalt in Gewichts-% |
|---|---|
| Wasser | 85,0 |
| Hydroxyectoin | 1,0 |
| Fettgrundlage | 10,3 |
| Mehrwertige Alkohole | 3,2 |
| Feste Verdickungsmittel | 0,3 |
| Konservierungsstoffe | 0,2 |

### Beispiel 3

### W/O Emulsion :

| Inhaltstoff | Gehalt in Gewichts-% |
|---|---|
| Wasser | 85,0 |
| Ectoin / Hydroxyectoin 1:1 | 1,0 |
| Fettgrundlage | 10,3 |
| Mehrwertige Alkohole | 3,2 |
| Feste Verdickungsmittel | 0,3 |
| Konservierungsstoffe | 0,2 |

### Beispiel 4

### Creme:

| Inhaltstoff | Gehalt in Gewichts-% |
|---|---|
| Wasser | 61,7 |
| Ectoin | 0,5 |
| Fettgrundlage | 32,8 |
| Mehrwertige Alkohole | 4,0 |
| Feste Verdickungsmittel | 0,5 |
| Konservierungsstoffe | 0,5 |

### Beispiel 5

### Creme:

| Inhaltstoff | Gehalt in Gewichts-% |
|---|---|
| Wasser | 61,7 |
| Hydroxyectoin | 0,5 |
| Fettgrundlage | 32,8 |
| Mehrwertige Alkohole | 4,0 |
| Feste Verdickungsmittel | 0,5 |
| Konservierungsstoffe | 0,5 |

### Beispiel 6

### Creme:

| Inhaltstoff | Gehalt in Gewichts-% |
|---|---|
| Wasser | 61,5 |
| Ectoin / Hydroxyectoin 1:1 | 1,0 |
| Fettgrundlage | 32,5 |
| Mehrwertige Alkohole | 4,0 |
| Feste Verdickungsmittel | 0,5 |
| Konservierungsstoffe | 0,5 |

## Patentansprüche

1. Verwendung von Ectoin, Hydroxyectoin, deren pharmakologisch verträglichen Salzen und/oder Verbindungen, die sich durch strukturelle Abweichungen der funktionellen Gruppen oder der Substituenten vom Ectoin oder Hydroxyectoin unterscheiden, zur Herstellung von dermatologischen Zubereitungen zur topischen Behandlung von Neurodermitis.

2. Verwendung nach Anspruch 1, **dadurch gekennzeichnet, dass** in der Verbindung die Hydroxylgruppe des Hydroxyectoins durch eine Alkoxylgruppe ersetzt ist, die mit einer gesättigten oder ungesättigten, geradkettigen oder verzweigten C¹ bis C⁴-Alkylgruppe gebildet ist.

3. Verwendung nach Anspruch 1, **dadurch gekennzeichnet, dass** in der Verbindung die Hydroxylgruppe des Hydroxyectoins mit einer C¹ bis C⁴-Carbonsäure verestert ist.

4. Verwendung nach Anspruch 1, **dadurch gekennzeichnet, dass** in der Verbindung die Carbonsäurefunktion des Ectoins oder Hydroxyectoins durch eine Carbonsäureamidfunktion substituiert ist, wobei das Amid gesättigte oder ungesättigte, geradkettige oder verzweigte C¹ bis C⁴-Alkylgruppen am Stickstoffatom aufweisen kann.

5. Verwendung nach Anspruch 1, **dadurch gekennzeichnet, dass** in der Verbindung die Carbonsäurefunktion des Ectoins oder Hydroxyectoins durch eine Carbonsäureesterfunktion substituiert ist, die mit einem C¹ bis C⁴-Alkohol gebildet ist.

6. Verwendung nach einem der Ansprüche 1 bis 5, **dadurch gekennzeichnet, dass** die dermatologische Zubereitung eine aus üblichen Hilfsstoffen zusammengesetzte Tinktur, Lotion, O/W-Emulsion, W/O-Emulsion, Creme, Salbe oder ein Hydrogel oder Spray ist.

7. Verwendung nach einem der Ansprüche 1 bis 6, **dadurch gekennzeichnet, dass** die dermatologische Zubereitung eine Salbe, Creme oder Lotion ist, die wirkstoffhaltige flexible Liposomen aufweist.

8. Verwendung nach einem der Ansprüche 1 bis 7, **dadurch gekennzeichnet, dass** die dermatologische Zubereitung eine Salbe, Creme oder Lotion ist, die mikrosomal verkapselten Wirkstoff enthält.

9. Dermatologische Zubereitung, enthaltend Ectoin, Hydroxyectoin, ein pharmakologisch verträgliches Salz des Ectoins oder Hydroxyectoins und/oder eine Verbindung, die sich durch strukturelle Abweichungen der funktionellen Gruppen oder der Substituenten vom Ectoin oder Hydroxyectoin unterscheidet, zur verwendung bei der topischen Behandlung von Neurodermitis.

10. Dermatologische Zubereitung nach Anspruch 9, **dadurch gekennzeichnet, dass** in der Verbindung die Hydroxylgruppe des Hydroxyectoins durch eine Alkoxylgruppe ersetzt ist, die mit einer gesättigten oder ungesättigten, geradkettigen oder verzweigten C¹ bis C⁴-Alkylgruppe gebildet ist.

11. Dermatologische Zubereitung nach Anspruch 9, **dadurch gekennzeichnet, dass** in der Verbindung die Hydroxylgruppe des Hydroxyectoins mit einer C¹ bis C⁴-Carbonsäure verestert ist.

12. Dermatologische Zubereitung nach Anspruch 9, **dadurch gekennzeichnet, dass** in der Verbindung die Carbonsäurefunktion des Ectoins oder Hydroxyectoins durch eine Carbonsäureamidfunktion substituiert ist, wobei das Amid gesättigte oder ungesättigte, geradkettige oder verzweigte C¹ bis C⁴-Alkylgruppen am Stickstoffatom aufweisen kann.

13. Dermatologische Zubereitung nach Anspruch 9, **dadurch gekennzeichnet, dass** in der Verbindung die Carbonsäurefunktion des Ectoins oder Hydroxyectoins durch eine Carbonsäureesterfunktion substituiert ist, die mit einem C¹ bis C⁴-Alkohol gebildet ist.

14. Dermatologische Zubereitung nach einem der Ansprüche 9 bis 13, **dadurch gekennzeichnet, dass** die dermatologische Zubereitung eine Salbe, Creme oder Lotion ist, die wirkstoffhaltige flexible Liposomen aufweist.

15. Dermatologische Zubereitung nach einem der Ansprüche 9 bis 14, **dadurch gekennzeichnet, dass** die dermatologische Zubereitung eine Salbe, Creme oder Lotion ist, die mikrosomal verkapselten Wirkstoff enthält.

## Claims

1. Use of ectoine, hydroxyectoine, their pharmacologically compatible salts and/or compounds showing structural differences of the functional groups or substituents to ectoine or hydroxyectoine, for the production of dermatological preparations for the topical treatment of neurodermatitis.

2. Use according to claim 1, **characterized in that** in the compound the hydroxyl group of hydroxyectoine is substituted by an alkoxy group that is formed with a saturated or unsaturated, straight-chain or branched C¹ to C⁴ alkyl group.

3. Use according to claim 1, **characterized in that** in the compound the hydroxyl group of hydroxyectoine forms an ester with a C¹ to C⁴ carboxylic acid.

4. Use according to claim 1, **characterized in that** in the compound the carboxyl group of ectoine or hydroxyectoine is substituted by an amide group of a carboxylic acid, wherein the amide comprises saturated or unsaturated, straight-chain or branched C¹ to C⁴ alkyl groups at the nitrogen atom.

5. Use according to claim 1, **characterized in that** in the compound the carboxyl group of ectoine or hydroxyectoine is substituted by an ester group of a carboxylic acid formed with a C¹ to C⁴ alcohol.

6. Use according to any of claims 1 to 5, **characterized in that** the dermatological preparation is a tincture, lotion, O/W emulsion, W/O emulsion, cream, ointment or a hydrogel or spray composed of customary auxiliary substances.

7. Use according to any of claims 1 to 6, **characterized in that** the dermatological preparation is an ointment, cream or lotion comprising active-agent containing flexible liposomes.

8. Use according to any of claims 1 to 7, **characterized in that** the dermatological preparation is an ointment, cream or lotion containing microsomally capsuled active-agent.

9. Dermatological preparation comprising ectoine, hydroxyectoine, a pharmacologically compatible salt of ectoine or hydroxyectoine and/or a compound that shows structural differences of the functional groups or substituents to ectoine or hydroxyectoine, for use in the topical treatment of neurodermatitis.

10. Dermatological preparation according to claim 9, **characterized in that** in the compound the hydroxyl group of hydroxyectoine is substituted by an alkoxy group that is formed with a saturated or unsaturated, straight-chain or branched C¹ to C⁴ alkyl group.

11. Dermatological preparation according to claim 9, **characterized in that** in the compound the hydroxyl group of hydroxyectoine forms an ester with a C¹ to C⁴ carboxylic acid.

12. Dermatological preparation according to claim 9, **characterized in that** in the compound the carboxyl group of ectoine or hydroxyectoine is substituted by an amide group of a carboxylic acid, wherein the amide comprises saturated or unsaturated, straight-chain or branched C¹ to C⁴ alkyl groups at the nitrogen atom.

13. Dermatological preparation according to claim 9, **characterized in that** in the compound the carboxyl group of ectoine or hydrokyectoine is substituted by an ester group of a carboxylic acid formed with a C¹ to C⁴ alcohol.

14. Dermatological preparation according to any of claims 9 to 13, **characterized in that** the dermatological preparation is an ointment, cream or lotion comprising active-agent containing flexible liposomes.

15. Dermatological preparation according to any of claims 9 to 14, **characterized in that** the dermatological preparation is an ointment, cream or lotion containing microsomally capsuled active-agent.

## Revendications

1. Utilisation d'ectoïne, d'hydroxyectoïne, de leurs sels pharmacologiquement acceptables et/ou de composés qui se distinguent de l'ectoïne ou de l'hydroxyectoïne par des modifications de structure des groupes fonctionnels ou des substituants, pour fabriquer des préparations dermatologiques destinées au traitement topique de la neurodermite.

2. Utilisation selon la revendication 1, **caractérisée en ce que**, dans le composé, le groupe hydroxyle de l'hydroxyectoïne est remplacé par un groupe alcoxyle, qui est formé avec un groupe alkyle en C¹ à C⁴ saturé ou insaturé, à chaîne droite ou ramifiée.

3. Utilisation selon la revendication 1, **caracterisée en ce que**, dans le composé, le groupe hydroxyle de l'hydroxyeotoïne est estérifié par un acide carboxylique en C¹ à C⁴.

4. Utilisation selon la revendication 1, **caractérisée en ce que**, dans le composé, la fonction acide carboxylique de l'ectoïne ou de l'hydroxyectoïne est substituée par une fonction carboxamide, l'amide pouvant présenter sur l'atome d'azote des groupes alkyle en C¹ à C⁴ saturés ou insaturés, à chaîne droite ou ramifiée.

5. Utilisation selon la revendication 1, **caractérisée en ce que**, dans le ccmposé, la fonction acide carboxylique de l'ectoïne ou de l'hydroxyectoïne est substituée par une fonction ester d'acide carboxylique, qui est formée avec un alcool en C¹ à C⁴.

6. Utilisation selon l'une des revendications 1 à 5, **caractérisée en ce que** la préparation pharmaceutique est une teinture, une lotion, une émulsion de type aqueux, une émulsion de type huileux, une crème, une pommade, ou un hydrogel ou un spray, constitué d'adjuvants usuels.

7. Utilisation selon l'une des revendications 1 à 6, **caractérisée en ce que** la préparation dermatologique est une pommade, une crème ou une lotion qui présente des liposomes flexibles contenant un principe actif.

8. Utilisation selon l'une des revendications 1 à 7, **caractérisée en ce que** la préparation dermatologique est une pommade, une crème ou une lotion, qui contient un principe actif encapsulé dans des microsomes.

9. Préparation dermatologique contenant de l'ectoïne, de l'hydroxyectoïne, un sel pharmacologiquement compatible de l'ectoïne ou de l'hydroxyectoïne et/ou un composé qui se distingue de l'ectoïne ou de l'hydroxyectoïne par des modifications de structure des groupes fonctionnels ou des substituants, pour une utilisation dans le cadre du traitement topique de la neurodermite.

10. Préparation dermatologique selon la revendication 9, **caractérisée en ce que**, dans le composé, le groupe hydroxyle de l'hydroxyectoïne est remplacé par un groupe alcoxyle, qui est formé avec un groupe alkyle en C¹ à C⁴ saturé ou insaturé, à chaîne droite ou ramifiée.

11. Préparation dermatologique selon la revendication 9, **caractérisée en ce que**, dans le composé, le groupe hydroxyle de l'hydroxyectoïne est estérifié par un acide carboxylique en C¹ à C⁴.

12. Préparation dermatologique selon la revendication 9, **caractérisée en ce que**, dans le composé, la fonction acide carboxylique de l'ectoïne ou de l'hydroxyectoïne est substituée par une fonction carboxamide, l'amide pouvant présenter sur l'atome d'azote des groupes alkyle en C¹ à C⁴ saturés ou insaturés, à chaîne droite ou ramifiée.

13. Préparation dermatologique selon la revendication 9, **caractérisée en ce que**, dans le composé, la fonction acide carboxylique de l'ectoïne ou de l'hydroxyectoïne est substituée par une jonction ester d'acide carboxylique, qui est formée avec un alcool en C¹ à C⁴.

14. Préparation dermatologique selon l'une des revendications 9 à 13, **caractérisée en ce que** la préparation dermatologique est une pommade, une crème ou une lotion, qui comprend des liposomes flexibles contenant un principe actif.

15. Préparation dermatologique selon l'une des revendications 9 à 14, **caractérisée en ce que** la préparation dermatologique est une pommade, une crème ou une lotion, qui continent un principe actif encapsulé dans des microsomes.
